# EUROPEAN PATENT APPLICATION

(11) **EP 1 623 709 A1**
(43) Date of publication of application: **08.02.2006**
(21) Application number: 04729765.0
(22) Date of filing: 27.04.2004
(51) Int. Cl.: A61K 31/5377, A61P 19/02, A61P 29/00, C07D 261/14

(54) **REMEDY FOR RHEUMATOID ARTHRITIS**

(30) Priority: 30.04.2003 JP 2003125523
(71) Applicant: Dainippon Sumitomo Pharma Co., Ltd., Osaka 541-8510 (JP)
(72) Inventor: TAKADA, Yoshihiro, Enoki-cho, Suita-shi, Osaka (JP); KANEKO, Munekiyo, Kyobashi 1-chome, Chuo-ku, Tokyo (JP); TAGASHIRA, Rie, Enoki-cho, Suita-shi, Osaka (JP)
(74) Representative: Duckett, Anthony Joseph
(86) International application number: PCT/JP2004/006105
(87) International publication number: WO 2004/096230

(57) **Abstract**

It is intended to provide a pharmaceutical composition for oral use which comprises SMP-114 or its pharmaceutically acceptable salt as the active ingredient, is designed so as to give an AUC₀₋₂₄ level of at least about 2.7 µg·hr/mL and aims at reduction of chronic inflammation, reduction of structural damage, reduction of progress of joint deformity or improving physical functions in patients with rheumatoid arthritis, whereby SMP-114 or its pharmaceutically acceptable salt is administered in a dose of from about 20 to 120 mg per day. Because of having excellent effects of ameliorating immunopathy and chronic inflammation, this composition is useful and clinically applicable as a medicament for treating or preventing rheumatoid arthritis with little side effects.

## Description

### TECHNICAL FIELD

The present invention relates to a pharmaceutical composition useful to patients with rheumatoid arthritis which comprises SMP-114 or its pharmaceutically acceptable salt as the active ingredient.

### BACKGROUD ART

Rheumatoid arthritis is a cryptogenic systemic inflammatory disease that causes systemic-arthritis as a main symptom and injures many organs. It proceeds chronically while repeating remission and exasperation. When neglected without treatment, it causes joint damage and deformity, damages physical functions, and can endanger the life of a patient in some cases. However, it is considered that currently medical science permits neither complete cure nor prevention of rheumatoid arthritis. Therefore, a therapeutic purpose to be pursued at the present is to improve the physical quality, mental quality and quality of life (QOL) of a patient by preventing the occurrence or progress of an irreversible change by carrying out early diagnosis, starting aggresive treatment in the early stage of the disease and suppressing rheumatic inflammation as quickly as possible and as much as possible.

Main drugs used for treating rheumatoid arthritis are nonsteroidal anti-inflammatory drugs (NSAIDs), disease modifying antirheumatic drugs (DMARDs) and adrenocortico-steroid drugs. NSAIDs are drugs used at first in patients with chronic rheumatoid arthritis. Although they bring about analgesic effect immediately, they exhibit anti-inflammatory effect after 1 to 2 weeks. NSAIDs are effective in reducing inflammation but are not effective in impeding the progress of rheumatic inflammation or preventing joint damage. In addition, many of the side effects of NSAIDs cannot be separated from their pharmacological action (cyclooxygenase-1 inhibitory activity), so that gastrointestinal disorders and the like often disturb the treatment. The term "DMARDs" is a general term for drugs for introducing remission by reducing rheumatic inflammation by ameliorating immunopathy due to rheumatoid arthritis. DMARDs generally have a slow onset of action and exhibit their continuous effect for a long period of time. DMARDs have no analgesic effect and are used in combination with NSAIDs having an immediate action or the adrenocorticosteroid drugs at the start of their administration. On the other hand, they are known to be disadvantageous in that they exhibit serious side effects to cause myelo-suppression, nephritis, interstitial pneumonia or the like in rare cases and that their optimum dosage varies depending on individuals. In addition, continuous administration of conventional DMARDs often results in acquisition of tolerance (escape phenomenon) and their effect diminishes in 2 to 3 years, so that the administration is often discontinued. The adrenocorticosteroid drugs suppress rheumatic inflammation rapidly and surely, and improve QOL of a patient with chronic rheumatoid arthritis though the improvement continues for only a short period of time. They, however, have many disadvantages such as the diminishment of their efficacy by long-term continuous use, withdrawal difficulty (rebound and withdrawal syndrome), the impossibility of preventing the progress of joint damage, and the exhibition of serious side effects including the induction of infectious diseases, adrenocortical insufficiency, gastrointestinal disorders, osteoporosis and the like

On the other hand, it is known that 3-[(1S)-1-(2-fluorobiphenyl-4-yl)ethyl]-5-{[amino(morpholin-4-yl)methylene]aminolisoxazole (hereinafter referred to also as "compound (I)" or "SMP-114") is useful as, for example, an excellent medicament for treating autoimmune diseases, inflammatory diseases and the like. That is, it has been reported that SMP-114 has antiarthritic effect on rats with adjuvant arthritis and mice with collagen arthritis, which are typical model animals for rheumatoid arthritis (see, for example, Shuzou Tagashira "Inflammation•Regeneration", Vol. 21, No. 4, p. 472 (2001); F. Nishikaku, "Annals of the Rheumatic Diseases", vol. 60 supplement 1, p. 159 (2001); and F. Nishikaku, "Annals of the Rheumatic Diseases", vol. 61 supplement 1, p. 194 (2002). Formula 1:

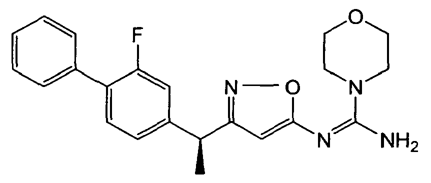

However, the pharmacokinetics, effective dose and the like of SMP-114 in the case of its administration to humans have not been specifically known at all.

### DISCLOSURE OF THE INVENTION

A medicament for treating autoimmune diseases such as rheumatoid arthritis should be clearly effective against chronic inflammation that causes tissue damage. For a fundamental therapy, it is important that the medicament also has inhibitory effect on the abnormality of the immune system which is responsible for the diseases. In addition, the medicament for treating the diseases is required to have little side effects because its long-term administration is often necessary.

In the past, there were a large number of proposed compounds that were expected to have clinical usefulness as candidates for medicaments for treating autoimmune diseases, on the basis of the results of efficacy tests using animal models. However, the development of many of these compounds was discontinued because they had side effects and/or insufficient efficacy in clinical studies.

The present invention is intended to provide a pharmaceutical composition that has excellent effects of ameliorating immunopathy and chronic inflammation and hence is useful and clinically applicable as a medicament for treating or preventing rheumatoid arthritis with little side effects.

The present inventors earnestly investigated in order to solve the above problems. An efficacy test on rat disease models was carried out by the use of SMP-114 to find that AUC₀₋₂₄ (indicating the 0-24 hours value of area under the serum concentration-time curve) for the exhibition of efficacy was 2711 to 3582 ng·hr/mL. In addition, as a result of a multiple dose study in adult subjects, the present inventors found that SMP-114 should be administered once a day in a dose of at least about 20 mg in order to attain the above AUC₀₋₂₄ level for the exhibition of efficacy in humans. The present inventors also found that SMP-114 is satisfactory in tolerance even when repeatedly administered once a day to human in a dose of about 120 mg. On the basis of these results, the present inventors found the suitable usage and dose of SMP-114 which bring about a marked inhibitory effect on rheumatic inflammation and the like with little side effects, whereby the present invention has been accomplished.

That is, the present invention is as follows.
[1] A pharmaceutical composition for oral use which comprises 3-[(1S)-1-(2-fluorobiphenyl-4-yl)ethyl]-5-{[amino(morpholin-4-yl)methylene]amino}isoxazole (SMP-114) or its pharmaceutically acceptable salt as the active ingredient, is designed so as to give an AUC₀₋₂₄ level of at least about 2.7 µg·hr/mL and aims at reduction of chronic inflammation, reduction of structural damage, reduction of progress of joint deformity or improving physical functions in patients with rheumatoid arthritis, whereby SMP-114 or its pharmaceutically acceptable salt is administered in a dose of from about 20 to 120 mg per day.
[2] A pharmaceutical composition for oral use which comprises SMP-114 or its pharmaceutically acceptable salt as the active ingredient, is designed so as to give an AUC₀₋₂₄ level of at least about 3.6 µg·hr/mL and aims at reduction of chronic inflammation, reduction of structural damage, reduction of progress of joint deformity or improving physical functions in patients with rheumatoid arthritis, whereby SMP-114 or its pharmaceutically acceptable salt is administered in a dose of from about 20 to 120 mg per day.
[3] The pharmaceutical composition according to the item [1] or [2], wherein the dose per day is about 40 to 120 mg.
[4] The pharmaceutical composition according to any one of the items [1] to [3], wherein the dose per day is about 40 mg, about 80 mg or about 120 mg.
[5] The pharmaceutical composition according to any one of the items [1] to [3], wherein the dose per day is about 120 mg.
[6] The pharmaceutical composition according to any one of the items [1] to [5], which is intended to be administered to patients with rheumatoid arthritis who have not been treated with disease modifying antirheumatic drugs (DMARDs) other than SMP-114 or have been treated with one or two DMARDs other than SMP-114.
[7] The pharmaceutical composition according to any one of the items [1] to [6], which is intended to be administered to patients with rheumatoid arthritis to whom disease modifying antirheumatic drugs (DMARDs) other than SMP-114 cannot be administered because of their side effects, or for whom DMARDs other than SMP-114 are not or insufficiently effective.
[8] The pharmaceutical composition according to any one of the items [1] to [7], which is intended to be administered to patients with rheumatoid arthritis who cannot take DMARDs other than SMP-114 because of their liver disorder, renal disorder or gastrointestinal disorder.
[9] The pharmaceutical composition according to any one of the items [1] to [8], which is intended to be administered to patients with rheumatoid arthritis who cannot take methotrexate or leflunomide because of their liver disorder, renal disorder or gastrointestinal disorder.
[10] The pharmaceutical composition according to any one of the items [1] to [9], which is intended to be administered to patients with rheumatoid arthritis who cannot expect the sufficient effect of biologicals because of, for example, the production of antibodies against said biologicals.
[11] The pharmaceutical composition according to any one of the items [1] to [10], which is intended to be administered to patients with rheumatoid arthritis who are liable to suffer infectious diseases.
[12] The pharmaceutical composition according to any one of the items [1] to [11], which is intended to be administered to patients with rheumatoid arthritis having a physician's global assessment of less than 63 as a VAS (visual analogue scale) score.
[13] The pharmaceutical composition according to any one of the items [1] to [12], which is intended to be administered to patients with rheumatoid arthritis having a patient's assessment of pain of less than 67 as a VAS (visual analogue scale) score.
[14] A method for reduction of chronic inflammation, reduction of structural damage, reduction of progress of joint deformity or improving physical functions in patients with rheumatoid arthritis, which comprises orally administering SMP-114 or its pharmaceutically acceptable salt as an active ingredient in a dose of from about 20 to 120 mg per day so that an AUC₀₋₂₄ level of at least about 2.7 µg·hr/mL may be given.
[15] A method for reduction of chronic inflammation, reduction of structural damage, reduction of progress of joint deformity or improving physical functions in patients with rheumatoid arthritis, which comprises orally administering SMP-114 or its pharmaceutically acceptable salt as an active ingredient in a dose of from about 20 to 120 mg per day so that an AUC₀₋₂₄ level of at least about 3.6 µg•hr/mL may be given.
[16] The method according to the item [14] or the item [15], wherein the dose per day is about 40 mg, about 80 mg or about 120 mg.
[17] The method according to the item [14] or the item [15], wherein the dose per day is about 120 mg.
[18] Use of SMP-114 or its pharmaceutically acceptable salt for the preparation of a pharmaceutical composition which is useful for reduction of chronic inflammation, reduction of structural damage, reduction of progress of joint deformity or improving physical functions in patients with rheumatoid arthritis, and satisfies the following conditions (1) and (2):
   (1) the pharmaceutical composition is prepared so that SMP-114 or its pharmaceutically acceptable salt may be orally administered as the active ingredient in a dose of from about 20 to 120 mg per day, and
   (2) the pharmaceutical composition is designed so as to give an AUC₀₋₂₄ level of SMP-114 of at least about 2.7 µg·hr/mL.
[19] Use according to the item [18], wherein the dose per day is about 40 mg, about 80 mg or about 120 mg.
[20] Use according to the item [18], wherein the dose per day is about 120 mg.
[21] A pharmaceutical composition comprising SMP-114 or its pharmaceutically acceptable salt as the active ingredient and aiming at reduction of chronic inflammation, reduction of structural damage, reduction of progress of joint deformity or improving physical functions in patients with rheumatoid arthritis, which is to be administered to patients with rheumatoid arthritis who have not been treated with disease modifying antirheumatic drugs (DMARDs) other than SMP-114 or have been treated with one or two DMARDs other than SMP-114.
[22] The pharmaceutical composition according to the item [21], wherein the patients have been treated with one or two disease modifying antirheumatic drugs (DMARDs) other than SMP-114.
[23] A pharmaceutical composition comprising SMP-114 or its pharmaceutically acceptable salt as the active ingredient and aiming at reduction of chronic inflammation, reduction of structural damage, reduction of progress of joint deformity or improving physical functions in patients with rheumatoid arthritis, which is to be administered to patients with rheumatoid arthritis having a physician's global assessment of less than 63 as a VAS (visual analogue scale) score.
[24] A pharmaceutical composition comprising SMP-114 or its pharmaceutically acceptable salt as the active ingredient and aiming at reduction of chronic inflammation, reduction of structural damage, reduction of progress of joint deformity or improving physical functions in patients with rheumatoid arthritis, which is to be administered to patients with rheumatoid arthritis having a patient's assessment of pain of less than 67 as a VAS (visual analogue scale) score.

### BEST MODE FOR CARRYING OUT THE INVENTION

The present invention is explained below in further detail.

SMP-114 used in the present invention may be used either in itself or in the form of a pharmaceutically acceptable salt. As the pharmaceutically acceptable salt of SMP-114, acid addition salts are exemplified. The acid addition salts include, for example, inorganic acid salts such as hydrochloride, hydrobromide, sulfate, hydroiodide, nitrate, phosphate, etc.; and organic acid salts such as citrate, oxalate, acetate, formate, propionate, benzoate, trifluoroacetate, fumarate, maleate, tartrate, aspartate, glutamate, methanesulfonate, benzenesulfonate, camphorsulfonate, etc. SMP-114 itself (free form) is preferable.

The "SMP-114 or its pharmaceutically acceptable salt" in the present invention includes those in the form of a solvate (e.g. a hydrate or an alcoholate) or in any crystal form.

SMP-114 used in the present invention may be produced, for example, by the process disclosed in the specification of Japanese Patent No. 3237608 (corresponding U.S. Patent: US 6,100,260) or the specification of Japanese Patent No. 3244672, and can have a plurality of crystal forms. A preferable example thereof is β-type crystals of SMP-114, namely, crystalline SMP-114 that shows main peaks at angles of diffraction (2θ) of 6.1°, 14.1°, 16.0°, 18.5°, 20.0° and 25.4° in powder X-ray diffraction. Further, the β-type crystals of SMP-114 may be produced by the process disclosed in International Publication No. WO02/092094 pamphlet (corresponding EP Patent: EP 1374871 A1).

The form of the "pharmaceutical composition for oral use" in the present invention includes, for example, tablets, capsules, pills, granules, powders, solutions, syrups and suspensions. These pharmaceuticals are prepared by conventional techniques and may contain conventional acceptable carriers, excipients, binders, stabilizers and the like. The pharmaceutical composition for oral use comprising SMP-114 or its pharmaceutically acceptable salt may be produced, for example, by any of the various processes disclosed in International Publication No. WO02/092094 pamphlet.

As the unit dosage form of the pharmaceutical composition for oral use of the present invention, there may be used a pharmaceutical composition comprising about 10 to 120 mg of SMP-114 or its pharmaceutically acceptable salt (in case of the SMP-114 salt, based on not the acid addition salt form of SMP-114 but SMP-114 itself of the acid addition salt form). An example thereof is a pharmaceutical composition in a unit dosage form comprising 10, 20, 30, 40, 50, 60, 70, 80, 90, 100, 110 or 120 mg of SMP-114.

As to the frequency of administration of the pharmaceutical composition of the present invention, the composition may be administered in one portion or several portions per day. The composition is preferably administered once a day. As to the dose of the pharmaceutical composition of the present invention, SMP-114 or its pharmaceutically acceptable salt may be administered as the active ingredient in a dose of from about 20 to 120 mg (in case of the SMP-114 salt, based on not the acid addition salt form of SMP-114 but SMP-114 itself of the acid addition salt form) per day as described above. Specifically, SMP-114 or its pharmaceutically acceptable salt may be administered, for example, in a dose of about 20 mg, about 30 mg, about 40 mg, about 50 mg , about 60 mg, about 70 mg, about 80 mg, about 90 mg, about 100 mg, about 110 mg or about 120 mg per day, preferably in a dose of about 40 mg, about 80 mg or about 120 mg per day, more preferably in a dose of about 120 mg per day. In addition, as to the dose of the pharmaceutical composition of the present invention, the composition may be administered also by a gradual increase method in which the administration is begun at a low dose and the dose is gradually increased by a definite amount each time so as to be in the above dose range of from about 20 to 120 mg.

The term "improving physical functions" used herein means improving daily-life motional capability by improving the symptoms of arthropathy (e.g. pain and expansion in joints caused by the chronic inflammation of synovial membrane) and reducting joint damage or deformity in patients with rheumatoid arthritis. The daily-life motional capability may be evaluated by indications such as putting-on and taking-off of clothes (dressing), standing, eating, walking, sanitation, extension, grip and action. Specifically, it may be evaluated by using HAQ (health assessment questionnaire) (Fries JF, et al., Arthritis Rheumatism, 23, p.137-145, 1980) to the patients.

The term "therapeutic effect" used herein means an effect judged by indications such as the reduction of chronic inflammation, joint damage or joint deformity or the improvement of physical functions in animals or patients suffering from rheumatoid arthritis. In addition, the following criterion of improvement established by the American College of Rheumatology. (ACR20; Felson Dt et al. The American College of Rheumatology preliminary definition of improvement in rheumatoid arthritis., Arthritis Rheum. 1995, 38:p.727-735; 1996 39:p.34-40) may be used.

When 20% or more improvement is observed in the items1) and 2) and 20% or more improvement is observed in at least three of the five items 3) to 7), the therapeutic effect is rated as "20% improvement on the criterion established by the American College of Rheumatology" (20% improvement on the ACR criterion).
1) Reduction of tender joint count.
2) Reduction of swollen joint count.
3) Patient's global assessment of disease activity.
4) Physician's global assessment of disease activity.
5) Patient's assessment of pain.
6) Patient's self-assessed physical functions.
7) CRP or erythrocyte sedimentation rate.

Similarly, when 50% or more improvement is observed in the items 1) and 2) and 50% or more improvement is observed in at least three of the five items 3) to 7), the therapeutic effect is rated as "50% improvement on the criterion established by the American College of Rheumatology" (50% improvement on the ACR criterion).

The term "AUC₀₋₂₄ level" used herein means the 0-24 hours value of area under the (SMP-114 concentration in serum) - time curve. The SMP-114 concentration in serum may be measured, for example, by an HPLC-fluorescence method employing the pretreatment and measuring conditions described in Example 1 or a LC-MS/MS method employing the pretreatment and measuring conditions described in Example 4.

The pharmaceutical composition of the present invention is particularly effective in treating the following patients with rheumatoid arthritis who are difficult to treat sufficiently by conventional treatments.

That is, the pharmaceutical composition of the present invention is particularly effective in treating (1) patients with rheumatoid arthritis to whom other DMARDs cannot be administered because of their side effects, or (2) patients with rheumatoid arthritis for whom other DMARDs are not or insufficiently effective.

The above-mentioned "DMARDs (disease modifying antirheumatic drugs)" include, for example, gold sodium thiomalate, auranofin, penicillamine, bucillamine, disodium robenzalit, salazosulfapyridine, actalit, methotrexate, myzoribin, sulfasalazine, azathioprine, hydroxychloroquine, chloroquine, leflunomide and SMP-114 used in the present invention.

The above term "other DMARDs" means DMARDs other than SMP-114. The side effects of the above-exemplified "other DMARDs" include, for example, liver disorder, renal disorder, gastrointestinal disorder, blood disorder and bone marrow disorder. More specifically, when methotrexate or leflunomide is selected as DMARD other than SMP-114, it is difficult for patients with rheumatoid arthritis having liver disorder, blood disorder or bone marrow disorder, to take methotrexate or leflunomide because of the side effects. SMP-114 having little side effects, however, is particularly effective in treating the above-mentioned patients with rheumatoid arthritis who cannot be treated with methotrexate or leflunomide.

Immunosuppressants are often used for treating rheumatoid arthritis, but they cannot be administered to patients with rheumatoid arthritis possessing decreased immunological function (for example, elderly patients with rheumatoid arthritis, and patients decreased in immunological function by the use of steroidal drugs or the like) because such patients are liable to suffer infectious diseases. Since SMP-114 has no common immunosuppressive effect, the pharmaceutical composition of the present invention is particularly effective in treating patients with rheumatoid arthritis who are liable to suffer infectious diseases.

Biological preparations (e.g. infliximab) are often used for treating rheumatoid arthritis, but an antibody (e.g. a neutralizing antibody) against the biological preparation is produced in some cases. In patients with rheumatoid arthritis having such an antibody (e.g. a neutralizing antibody) produced therein, the sufficient effect of the biological preparation cannot be expected. Therefore, the pharmaceutical composition of the present invention is particularly effective in treating patients with rheumatoid arthritis having such an antibody produced therein.

### EXAMPLES

The present invention is illustrated in further detail with reference to the following reference examples and working examples, which should not be construed as limiting the scope of the invention.

### Reference Example 1

### Preparation of tablets (20-mg tablets)

According to the following recipe, mannitol, corn starch and sodium croscarmelllose were charged into a fluidized-bed granulator and subjected to spray granulation by the use of a binding liquid obtained by dispersing and suspending a slightly water-soluble active ingredient in a water-soluble polymer binder solution, and the resulting granulation product was blended with magnesium stearate and the blend was compressed into tablets (20-mg tablets).

**Table 1**

| Component | Content (mg) |
|---|---|
| SMP-114 | 20 |
| Mannitol | 66 |
| Corn starch | 28 |
| Sodium croscarmelllose | 6 |
| Hydroxypropylmethyl cellulose | 4 |
| Magnesium stearate | 1 |
| Total | 125 mg |

As the above-mentioned SMP-114, its β-type crystals produced by the process disclosed in International Publication No. WO02/092094 pamphlet were used.

### Reference Example 2

### Preparation of tablets (40-mg tablets)

According to the following recipe, mannitol, corn starch and sodium croscarmelllose were charged into a fluidized-bed granulator and subjected to spray granulation by the use of a binding liquid obtained by dispersing and suspending a slightly water-soluble active ingredient in a water-soluble polymer binder solution, and the resulting granulation product was blended with magnesium stearate and the blend was compressed into tablets (40-mg tablets).

**Table 2**

| Component | Content (mg) |
|---|---|
| SMP-114 | 40 |
| Mannitol | 132 |
| Corn starch | 56 |
| Sodium croscarmelllose | 12 |
| Hydroxypropylmethyl cellulose | 8 |
| Magnesium stearate | 2 |
| Total | 250 mg |

As the above-mentioned SMP-114, its β-type crystals produced by the process disclosed in International Publication No. WO02/092094 pamphlet were used.

### Reference Example 3

### Preparation of film-coated tablets

The uncoated tablets prepared in Reference Example 1 were charged into Highcoater HCT30N (Freund Sangyo K.K.) and coated so as to have a coating film in an amount of 3 mg, to obtain film-coated tablets (20-mg tablets) having the following composition:

**Table 3**

| Component | Content (mg) |
|---|---|
| Uncoated tablets prepared in Reference Example 1 | 125 |
| Hydroxypropylmethyl cellulose | 2.13 |
| Macrogol 400 | 0.21 |
| Titanium oxide | 0.66 |
| Carnauba wax | trace |
| Total | 128 mg |

### Reference Example 4

### Preparation of a SMP-114 suspension

A suspension was prepared at the time of use and used, by the following steps 1 to 3.

### Step 1): Preparation of a solution for suspension

a) In a bottle containing 600 mg of a 1 : 5 mixture of HPMC (hydroxypropylmethyl cellulose) and D-mannitol as a suspending agent was gently placed 50 mL of purified water.
b) The bottle was capped and then subjected to ultrasonification in an ultrasonic bath for 10 minutes or more to dissolve the suspending agent completely, whereby a solution for suspension was prepared.

### Step 2): Preparation of a suspension

a) The whole solution for suspension prepared in step 1 was placed in each of bottles containing the active ingredient (SMP-114) in an amount of 10, 20, 40, 60, 80, 100, 120 or 140 mg, respectively, depending on dose. Each bottle was capped and its content was mixed by vigorous shaking by hand for 10 seconds or more.
b) The bottle was placed in an ultrasonic bath and subjected to ultrasonification for 10 minutes or more to disperse the active ingredient completely.
c) The content of the bottle was mixed again by vigorous shaking by hand for 10 seconds or more to become homogeneous, whereby a SMP-114 suspension was prepared.

### Step 3): Administration method

a) The SMP-114 suspension prepared in step 2 was mixed by shaking and administered orally and directly from the bottle.
b) In the empty bottle after the administration was placed 50 mL of purified water and the whole bottle was rinsed out by mixing the purified water by shaking. The washing solution was orally administered.

As the above-mentioned SMP-114, its β-type crystals produced by the process disclosed in International Publication No. WO02/092094 pamphlet were used.

In the following working examples, as tablets, 20-mg film-coated tablets obtained by the process described in Reference Example 3 or 10-mg film-coated tablets obtained by the same process were used. As suspensions, suspensions for the various doses, respectively, obtained by the process described in Reference Example 4 were used. As a placebo suspension, a suspension prepared in the same manner as in Reference Example 4 was used.

### Example 1

### AUC₀₋₂₄ level for the exhibition of efficacy in rats

The minimum effective amount of SMP-114 for the observation of its antiarthritic effect in an animal model was 2.5 mg/kg (once-α-day oral administration) in the case of rats with adjuvant arthritis (Shuzou Tagashira "Inflammation•Regeneration", Vol. 21, No. 4, p. 472 (2001)). It was 5 mg/kg (once-a-day oral administration) in the case of mice with collagen arthritis (F. Nishikaku, "Annals of the Rheumatic Diseases", vol. 61 supplement 1, p. 194 (2002)). On the basis of these results, it was considered that SMP-114 exhibits its efficacy at a dose of 5 mg/kg or more.

Next, a repeated oral administration toxicity test on Crj:CD(SD) rats aged 5 weeks was carried out for each of one month and six months (once-a-day oral administration in both cases). SMP-114 was repeatedly administered to male and female SD strain rats (10 rats per group) for one month in a dose of each of 10, 30, 100 and 300 mg/kg, and its subacute toxicity and the recovery by one month of the suspension of the administration were investigated. In addition, the time-course of SMP-114 concentration in serum after each of one run of the administration and 30 days of the repeated administration was investigated. Furthermore, SMP-114 was repeatedly administered to male and female SD strain rats (12 rats per group) for 6 months in a dose of each of 6, 20, 60 and 200 mg/kg, and its subacute toxicity and the recovery by three months of the suspension of the administration were investigated. In addition, the time-course of SMP-114 concentration in serum after each of one run of the administration, 3 months of the repeated administration and 6 months of the repeated administration was investigated. The SMP-114 concentration in serum was measured by an HPLC-fluorescence method (fluorescence wavelength: 319 nm) after extracting SMP-114 from each sample with diethyl ether under slight acidic conditions (pH 4.7).

When SMP-114 was administered to the rats in a dose of 10 mg/kg for one month, AUC₀₋₂₄ was 7164 ng·hr/mL. When SMP-114 was administered to the rats in a dose of 6 mg/kg for 6 months, AUC₀₋₂₄ was 3254 ng·hr/mL. Therefore, AUC₀₋₂₄ in the case of the administration of SMP-114 in a dose of 5 mg/kg was calculated to be 2711 to 3582 ng·hr/mL.

### Example 2

### AUC₀₋₂₄ level of the agent in its repeated administration to humans

A repeated administration test on healthy male Japanese of 20 to 40 years old was carried out. Using the SMP-114 tablets and placebo tablets, 80 mg of each of the placebo and SMP-114 was orally administered once a day for 7 days by a placebo controlled double-blind method (8 cases of SMP-114 administration and 2 cases of placebo administration: 10 subjects in total).

In the administration of 80 mg of SMP-114, the level of AUC₀₋₂₄ at a steady state was 15258.4 ng·hr/mL. In addition, the tolerance at this dose was judged satisfactory. The SMP-114 concentration in serum substantially reached the steady state when SMP-114 was administered 3 to 5 times. SMP-114 seemed to have no accumulative property.

The AUC₀₋₂₄ level for the exhibition of efficacy in rats described in Example 1 is 2711 to 3582 ng·hr/mL, and in order to attain this level in human beings, a dose of 14 to 19 mg/day is necessary according to calculation from the above-mentioned AUC₀₋₂₄ level (15258.4 ng•hr/mL), indicating that SMP-114 exhibits its efficacy in human beings at a dose of about 20 mg/day or more.

### Example 3

### Tolerance test by a single administration

(1) A single-administration test on healthy male Japanese (20 to 40 years old, body weight: less than 80 kg and not less than 50 kg, degree of corpulence: ± 20% or less) was carried out. Using the SMP-114 tablets and a placebo as control, 10, 20, 40, 80 or 120 mg of SMP-114 was administered by a single-blind method when the subjects were hungry (at each dose, 6 cases of SMP-114 administration and 2 cases of placebo administration: 40 subjects in total). As to items of evaluation related to safety, observations about the following were carried out: vital signs (body temperature, blood pressure•pulse rate and respiration rate), 12-induction electrocardiogram, clinical examinations [hematological examination, blood biochemical examination, endocrinological examinations (FT4, FT3 and TSH), serological examination (haptoglobin) and urinalysis], and harmful matters. As items of evaluation related to pharmacokinetics, the concentrations of SMP-114 and its metabolites in serum and urine were measured. Side effects undesirable with respect to tolerance were observed at none of the doses employed in the administration.
(2) A single-administration test on healthy male Caucasians (18 to 50 years old, BMI: 18 to 27 kg/m²) was carried out. Using the SMP-114 suspensions and the placebo suspension, 10, 20, 40, 60, 80, 100, 120 or 140 mg of each of the placebo and SMP-114 was orally administered by a placebo controlled double-blind method when the subjects were fasted (at each dose, 6 cases of SMP-114 administration and 3 cases of placebo administration: 72 subjects in total). As to items of evaluation related to safety, observations about the following were carried out: vital signs (body temperature, blood pressure•pulse rate and respiration rate), 12-lead electrocardiogram, clinical examinations [hematological examination, blood biochemical examination, endocrinological examinations (FT4, FT3 and TSH) and urinalysis], and harmful matters. As items of evaluation related to pharmacokinetics, the concentrations of SMP-114 and its metabolites in serum and urine were measured. Side effects undesirable with respect to tolerance was observed at none of the doses employed in the administration.

### Example 4

### Tolerance limit on SMP-114

A repeated administration test on healthy male Caucasians of 18 to 50 years old was carried out. Using the SMP-114 tablets and placebo tablets, 80 mg or 120 mg of each of the placebo and SMP-114 was orally administered once a day for 7 days by a placebo controlled double-blind method (at each dose, 8 cases of SMP-114 administration and 2 cases of placebo administration: 20 subjects in total).

Side effects undesirable with respect to tolerance were observed at neither of the doses of 80 mg and 120 mg. Therefore, SMP-114 was judged to be satisfactory in tolerance at a dose of about 120 mg or less.

The SMP-114 concentration in serum substantially reached a steady state when SMP-114 was administered 3 to 5 times. SMP-114 had no accumulative property. The level of AUC₀₋₂₄ at the steady state was 10574 ng•hr/mL in the 80 mg treated group and 16655 ng·hr/mL in the 120 mg treated group.

The SMP-114 concentration in serum was measured by a LC-MS/MS method by employing the following pretreatment and measuring conditions.

### 1. Pretreatment Procedure

Standard sample solutions for calibration curve, a QC sample and samples to be analyzed were pretreated as follows.
1) To each of the standard sample solutions for calibration curve, the QC sample and the samples to be analyzed was added 10 mL of an internal standard solution (d₅-SMP-114 5 ng/mL), and stirred.
2) To the resulting mixture were added 1 mL of 100 mmol/L ammonium acetate buffer (pH 4.0) and then 6 mL of diethyl ether, and stirred for 10 minutes, followed by centrifugation at 4°C and 3000 rpm for 10 minutes.
3) The supernatant was transferred to a glass tube and concentrated to dryness in a nitrogen stream (40°C) in an aluminum-blocked thermostatic chamber.
4) To the residue was added 120 µL of 50 vol% acetonitrile and the residue was redissolved with an ultrasonic bath and a Vortex mixer.
5) The solution obtained by the redissolution was transferred to a filter and centrifuged at 5000 rpm for 3 minutes.
6) The filtrate was transferred to an auto-sampler vial to obtain a sample solution for measurement.

### 2. Measuring Conditions

LC-MS/MS measurement was carried out under the following conditions. However, parameters (e.g. gas volume and collision voltage) whose optimum values were variable depending on the condition of a mass spectrometer were properly changed.

### 1. LC conditions

Mobile phase:
eluent A; 0.3 vol% acetic acid
eluent B; acetonitrile

Gradient table:

| Time (min.) | A (%) | B (%) |
|---|---|---|
| 0 | 50 | 50 |
| 5 | 0 | 100 |
| 5.1 | 50 | 50 |
| 15 | 50 | 50 |

Flow rate: 0.2 ml/min
Analytical column: Puresil C18, 2.1 mm i.d. × 150 mm, 5 mm (Waters)
Auto-sampler tray: 4°C
Column temperature: 35°C
Valve switching: 0-1.5 min. discard, 1.5-9.0 min. ion source for SMP-114, 9.0-15.0 min. discard
Injecting volume: 5 mL

### 2) MS/MS conditions

Ionization method: electro-ion spray method, positive mode
MS/MS mode: SRM (selected reaction monitoring)
Monitor ion: SMP-114 Q1 m/z 395.1 → Q3 m/z 199.0 (-25 eV) d₅-SMP-114 Q1 m/z 400.2 → Q3 m/z 204.0 (-25 eV)
Total scan time: 0.6 second (SMP-114 and d₅-SMP-114)
Ionization voltage: 4.5 kV for SMP-114
Sheath gas: 70 psi (nitrogen gas)
Auxiliary gas: 30 units (nitrogen gas)
Capillary tube temperature: 230°C
Collision gas: 2.1-2.2 mT (argon)
Multiplier: 1300 V

### Example 5

### Administration to patients with rheumatoid arthritis in an effective dose

SMP-114 is orally administered to patients with rheumatoid arthritis once a day after a meal. As to the dose, a placebo or a dose of 40 mg/day is employed (15 cases per group). The administration period is 24 weeks. SMP-114 is administered in a dose of 20 mg/day for the first 4 weeks and then in a dose of 40 mg/day for the subsequent 20 weeks. Twenty-four weeks after the start of the administration, the placebo group and the 40 mg/day group were compared with respect to the proportion of patients satisfying the requirement "20% improvement on the criterion established by the American College of Rheumatology", whereby the efficacy of the SMP-114 can be evaluated.

### Example 6

### Administration to patients with rheumatoid arthritis in an effective dose

### 1. Test method and 20% improvement rate on the ACR criterion

SMP-114 was orally administered once a day after a meal to patients with rheumatoid arthritis who had been treated with 1 to 5 DMARDs other than SMP-114 (to whom these DMARDs seemed to be non-administrable because of their side effects, or for whom these DMARDs seemed to be ineffective or insufficiently effective). As to the dose, a placebo, 40 mg/day, 80 mg/day and 120 mg/day were employed (about 50 cases per group). The administration period was 24 weeks, but patients in whom a specified standard of efficacy had not been reached were excluded at the 16th week (when 20% or more improvement was observed in any of tender joint count, swollen joint count and erythrocyte sedimentation rate, it was judged that the specified standard of efficacy had been reached). SMP-114 was continuously administered to patients in whom the standard had been reached, until the 24th week while keeping the test blind. Sixteen weeks after the start of the administration, the placebo group, 40 mg/day group, 80 mg/day group and 120 mg/day group were compared with respect to the proportion of patients satisfying the requirement "20% improvement on the criterion established by the American College of Rheumatology", whereby the efficacy of the SMP-114 was evaluated (in the present example, the following ratings were given on a visual analogue scale (VAS): 3) a patient's global assessment of disease activity, 4) a physician's global assessment of disease activity, and 5) a patient's assessment of pain, on the ACR criterion.).

As a result, it was found that the proportion of patients satisfying the requirement "20% improvement on the criterion established by the American College of Rheumatology" sixteen weeks after the start of the administration was higher in all the active groups than in the placebo group as follows when erythrocyte sedimentation rate was used as the item 7): the proportion values obtained for the placebo group, 40 mg/day group, 80 mg/day group and 120 mg/day group were 23.7, 38.2, 36.4 and 37.0%, respectively.

Also when CRP value was used as the item 7), the proportion was higher in all the active groups than in the placebo group as follows: the proportion values obtained for the placebo group, 40 mg/day group, 80 mg/day group and 120 mg/day group were 20.3, 38.2, 34.5 and 37.0%, respectively.

**Table 4 When erythrocyte sedimentation rate was used**

| | Placebo | 40 mg/day | 80 mg/day | 120 mg/day |
|---|---|---|---|---|
| Number of patients (*1) | 59 | 55 | 55 | 54 |
| Number of ACR20 responders (*2) | 14 | 21 | 20 | 20 |
| ACR20 improvement rate (3*) | 23.7 | 38.2 | 36.4 | 37.0 |

| | | | | |
|---|---|---|---|---|
| (*1) Number of patients: all patients treated at least once with the investigational drug | | | | |
| (*2) Number of ACR20 responders: the number of patients satisfying the requirement "20% improvement on the ACR criterion" | | | | |
| (*3) ACR20 improvement rate: the proportion (%) of patients satisfying the requirement "20% improvement on the ACR criterion" | | | | |

**Table 5 When CRP was used**

| | Placebo | 40 mg/day | 80 mg/day | 120 mg/day |
|---|---|---|---|---|
| Number of patients (*1) | 59 | 55 | 55 | 54 |
| Number of ACR20 responders (*2) | 12 | 21 | 19 | 20 |
| ACR20 improvement rate (3*) | 20.3 | 38.2 | 34.5 | 37.0 |

| | | | | |
|---|---|---|---|---|
| (*1) Number of patients: all patients treated at least once with the investigational drug | | | | |
| (*2) Number of ACR20 responders: the number of patients satisfying the requirement "20% improvement on the ACR criterion" | | | | |
| (*3) ACR20 improvement rate: the proportion (%) of patients satisfying the requirement "20% improvement on the ACR criterion" | | | | |

### 2. 50% Improvement rate on the ACR criterion

When the following improvements are observed on the above-mentioned criterion of improvement established by the American College of Rheumatology, it is judged that "50% improvement on the criterion established by the American College of Rheumatology" (50% improvement on the ACR criterion) has been achieved: 50% or more improvement is observed in the items 1) and 2) and 50% or more improvement is observed in at least three of the five items 3) to 7). The efficacy of SMP-114 may be evaluated on the basis of the proportion of patients satisfying the requirement "50% improvement on the criterion established by the American College of Rheumatology".

The proportion of patients satisfying the requirement "50% improvement on the criterion established by the American College of Rheumatology" sixteen weeks after the start of the administration was higher in all the active groups than in the placebo group as follows when erythrocyte sedimentation rate of the item 7) was used: the proportion values obtained for the placebo group, 40 mg/day group, 80 mg/day group and 120 mg/day group were 3.4, 10.9, 10.9 and 13.0%, respectively.

**Table 6**

| | Placebo | 40 mg/day | 80 mg/day | 120 mg/day |
|---|---|---|---|---|
| Number of patients (*1) | 59 | 55 | 55 | 54 |
| Number of ACR50 responders (*2) | 2 | 6 | 6 | 7 |
| ACR50 improvement rate (3*) | 3.4 | 10.9 | 10.9 | 13.0 |

| | | | | |
|---|---|---|---|---|
| (*1) Number of patients: all patients treated at least once with the investigational drug | | | | |
| (*2) Number of ACR50 responders: the number of patients satisfying the requirement "50% improvement on the ACR criterion" | | | | |
| (*3) ACR50 improvement rate: the proportion (%) of patients satisfying the requirement "50% improvement on the ACR criterion" | | | | |

### 3. ACR core set rating items

The efficacy of SMP-114 may be evaluated on the basis of improvement rates of the above-mentioned rating items on the criterion established by the American College of Rheumatology and QOL measure (SF-36) (Ware, JE. & Sherbourne CD., "The MOS 36-Item Short-Form Health Survey (SF-36); 1. Conceptual Framework and Item Selection.", Med Care 1981; 18:806). The improvement rates may be expressed as the rates of change calculated on the basis of the difference between values obtained 16 weeks after the start of the administration and initial values obtained before the start of the administration.

The improvement rates of the rating items 16 weeks after the start of the administration were as follows in the placebo group, 40 mg/day group, 80 mg/day group and 120 mg/day group. The improvement rates of, in particular, "patient's global assessment of disease activity" and "SF-36 physical scale" were significantly higher in all the active groups than in the placebo group.
1) Reduction of tender joint count
   20.2, 16.0, 27.6, 22.7% (p value: 0.667)
2) Reduction of swollen joint count
   21.6, 22.3, 18.7, 19.7% (p value: 0.985)
3) Patient's global assessment of disease activity
   2.1, 19.3, 20.5, 14.2% (p value: 0.043)
4) Physician's global assessment of disease activity
   11.9, 12.7, 21.5, 24.1% (p value: 0.308)
5) Patient's assessment of pain
   0.7, 17.1, 16.5, 17.1% (p value: 0.056)
6) Patient's self-assessed physical functions
   -4.7, 4.3, 10.6, -8.7% (p value: 0.119)
7)-1 CRP
   -134.4, -12.3, -26.4, -98.6 (p value: 0.089)
7)-2 Erythrocyte sedimentation rate
   -2.6, 3.2, 4.0, -7.5% (p value: 0.511)
   - SF-36 physical scale
      29.2, 33.3, 28.8, 30.2% (p value: 0.029)
   - SF-36 mental scale
      43.1, 43.7, 46.1, 44.8% (p value: 0.401)

### 4. Stratification analysis (1)

Stratification analysis was carried out by employing as an indication the history of treatment with disease modifying antirheumatic drugs (DMARDs) other than SMP-114, i.e., the number of the DMARDs other than SMP-114 which had been used. There are shown below the results of the analysis carried out by dividing patients into patients with rheumatoid arthritis who had been treated with one or two DMARDs other than SMP-114 (group A) and patients with rheumatoid arthritis who had been treated with 3 to 5 DMARDs other than SMP-114 (group B).

**Table 7 Stratification analysis: ACR20 improvement rate (%) dependent on the history of treatment with DMARDs**

| | Placebo | Total active groups (*) |
|---|---|---|
| Group A (DMARDs treatment history = 1- 2 drugs) | 19 (6/31 cases) | 44 (32/72 cases) |
| Group B (DMARDs treatment history = 3- 5 drugs) | 29 (8/28 cases) | 32 (29/92 cases) |

| | | |
|---|---|---|
| (*) The term "total active groups" means the sum of the three groups treated with the real drug, i.e., the 40 mg/day group, 80 mg/day group and 120 mg/day group; hereinafter the same applied. | | |

The above results reveal that the therapeutic effect of SMP-114 is more remarkable in the patients with rheumatoid arthritis treated with one or two DMARDs other than SMP-114 than in the patients with rheumatoid arthritis treated with 3 to 5 DMARDs other than SMP-114. That is, patients with rheumatoid arthritis to whom the pharmaceutical composition of the present invention is administered are preferably patients with rheumatoid arthritis who have been treated with two or less DMARDs other than SMP-114 (namely, who have not been treated with DMARDs or have been treated with one or two DMARDs) (such patients are considered as patients having a low resistance to treatment with DMARDs). It was found that a more marked therapeutic effect could be obtained in such patients.

### 5. Stratification analysis (2)

Stratification analysis was carried out by employing "physician's global assessment (VAS)" and "patient's assessment of pain (VAS)" as indications. (Felson Dt et al., Arthritis Rheum., 1995, 38:p.727-735).

The term "VAS (visual analog scale), 100 mm" means an indication obtained by the expression of rating from 0 on a line of 100 mm by an estimator. The rating is expressed as integers of 0 to 10. Although the length of the line may be other than 100 mm (for example, 200 mm), the term "VAS (visual analog scale)" used in the present specification means an indication obtained by giving a score in the range of 0 to 100.

"Physician's global assessment (VAS)" is an indication of a global assessment for disease activity given by a physician, and is given by assessment the condition of arthritis of a patient by the use of the VAS score (No disease activity: 0, very severe disease: 100).

"Patient's assessment of pain (VAS)" is an indication of an assessment of pain given by the patient, and is such that the patient himself (herself) assesses his (her) pain by the use of the VAS score (no pain: 0, maximum pain: 100).

The results of the differential analysis are shown below.

**Table 8 Stratification analysis: ACR20 improvement rate (%) dependent on "physician's global assessment (VAS)" score**

| | Placebo | Total active groups |
|---|---|---|
| "Physician's global assessment (VAS)" < 63 | 21 (6/28 cases) | 42 (31/74 cases) |
| "Physician's global assessment (VAS)" ≥ 63 | 26 (8/31 cases) | 33 (30/90 cases) |

**Table 9 Stratification analysis: ACR20 improvement rate (%) dependent on "patient's assessment of pain (VAS)" score**

| | Placebo | Total active groups |
|---|---|---|
| "Patient's assessment of pain (VAS)" < 67 | 26 (6/23 cases) | 50 (36/72 cases) |
| "Patient's assessment of pain (VAS)" ≥ 67 | 22 (8/36 cases) | 27 (25/92 cases) |

The above results reveals that the therapeutic effect of SMP-114 is remarkable in patients with rheumatoid arthritis having a "physician's global assessment (VAS)" < 63 or a "patient's assessment of pain (VAS)" < 67. That is, it was found that the pharmaceutical composition of the present invention gives more remarkable therapeutic effect to patients with rheumatoid arthritis who have been diagnosed as having a "physician's global assessment (VAS)" < 63 and/or a "patient's assessment of pain (VAS)" < 67 before the administration of the pharmaceutical composition.

### INDUSTRIAL APPLICABILITY

According to the present invention, it is possible to provide a medicament for treating rheumatoid arthritis with little side effects which improves the physical quality, mental quality and quality of life (QOL) of a patient by preventing the occurrence or progress of an irreversible change by suppressing rheumatic inflammation as quickly as possible and as much as possible.

## Claims

1. A pharmaceutical composition for oral use which comprises 3-[(1S)-1-(2-fluorobiphenyl-4-yl)ethyl]-5-{[amino(morpholin-4-yl)methylene]amino}isoxazole (SMP-114) or its pharmaceutically acceptable salt as the active ingredient, is designed so as to give an AUC₀₋₂₄ level of at least about 2.7 µg•hr/mL and aims at reduction of chronic inflammation, reduction of structural damage, reduction of progress of joint deformity or improving physical functions in patients with rheumatoid arthritis, whereby SMP-114 or its pharmaceutically acceptable salt is administered in a dose of from about 20 to 120 mg per day.

2. A pharmaceutical composition for oral use which comprises SMP-114 or its pharmaceutically acceptable salt as the active ingredient, is designed so as to give an AUC₀₋₂₄ level of at least about 3.6 µg·hr/mL and aims at reduction of chronic inflammation, reduction of structural damage, reduction of progress of joint deformity or improving physical functions in patients with rheumatoid arthritis, whereby SMP-114 or its pharmaceutically acceptable salt is administered in a dose of from about 20 to 120 mg per day.

3. The pharmaceutical composition according to claim 1 or 2, wherein the dose per day is about 40 to 120 mg.

4. The pharmaceutical composition according to any one of claims 1 to 3, wherein the dose per day is about 40 mg, about 80 mg or about 120 mg.

5. The pharmaceutical composition according to any one of claims 1 to 3, wherein the dose per day is about 120 mg.

6. The pharmaceutical composition according to any one of claims 1 to 5, which is intended to be administered to patients with rheumatoid arthritis who have not been treated with disease modifying antirheumatic drugs (DMARDs) other than SMP-114 or have been treated with one or two DMARDs other than SMP-114.

7. The pharmaceutical composition according to any one of claims 1 to 6, which is intended to be administered to patients with rheumatoid arthritis to whom disease modifying antirheumatic drugs (DMARDs) other than SMP-114 cannot be administered because of their side effects, or for whom DMARDs other than SMP-114 are not or insufficiently effective.

8. The pharmaceutical composition according to any one of claims 1 to 7, which is intended to be administered to patients with rheumatoid arthritis who cannot take DMARDs other than SMP-114 because of their liver disorder, renal disorder or gastrointestinal disorder.

9. The pharmaceutical composition according to any one of claims 1 to 8, which is intended to be administered to patients with rheumatoid arthritis who cannot take methotrexate or leflunomide because of their liver disorder, renal disorder or gastrointestinal disorder.

10. The pharmaceutical composition according to any one of claims 1 to 9, which is intended to be administered to patients with rheumatoid arthritis who cannot expect the sufficient effect of biologicals because of, for example, the production of antibodies against said biologicals.

11. The pharmaceutical composition according to any one of claims 1 to 10, which is intended to be administered to patients with rheumatoid arthritis who are liable to suffer infectious diseases.

12. The pharmaceutical composition according to any one of claims 1 to 11, which is intended to be administered to patients with rheumatoid arthritis having a physician's global assessment of less than 63 as a VAS (visual analogue scale) score.

13. The pharmaceutical composition according to any one of claims 1 to 12, which is intended to be administered to patients with rheumatoid arthritis having a patient's assessment of pain of less than 67 as a VAS (visual analogue scale) score.

14. A method for reduction of chronic inflammation, reduction of structural damage, reduction of progress of joint deformity or improving physical functions in patients with rheumatoid arthritis, which comprises orally administering SMP-114 or its pharmaceutically acceptable salt as an active ingredient in a dose of from about 20 to 120 mg per day so that an AUC₀₋₂₄ level of at least about 2.7 µg·hr/mL may be given.

15. A method for reduction of chronic inflammation, reduction of structural damage, reduction of progress of joint deformity or improving physical functions in patients with rheumatoid arthritis, which comprises orally administering SMP-114 or its pharmaceutically acceptable salt as an active ingredient in a dose of from about 20 to 120 mg per day so that an AUC₀₋₂₄ level of at least about 3.6 µg·hr/mL may be given.

16. The method according to claim 14 or 15, wherein the dose per day is about 40 mg, about 80 mg or about 120 mg.

17. The method according to claim 14 or 15, wherein the dose per day is about 120 mg.

18. Use of SMP-114 or its pharmaceutically acceptable salt for the preparation of a pharmaceutical composition which is useful for reduction of chronic inflammation, reduction of structural damage, reduction of progress of joint deformity or improving physical functions in patients with rheumatoid arthritis, and satisfies the following conditions (1) and (2):
(1) the pharmaceutical composition is prepared so that SMP-114 or its pharmaceutically acceptable salt may be orally administered as the active ingredient in a dose of from about 20 to 120 mg per day, and
(2) the pharmaceutical composition is designed so as to give an AUC₀₋₂₄ level of SMP-114 of at least about 2.7 µg•hr/mL.

19. Use according to claim 18, wherein the dose per day is about 40 mg, about 80 mg or about 120 mg.

20. Use according to claim 18, wherein the dose per day is about 120 mg.

21. A pharmaceutical composition comprising SMP-114 or its pharmaceutically acceptable salt as the active ingredient and aiming at reduction of chronic inflammation, reduction of structural damage, reduction of progress of joint deformity or improving physical functions in patients with rheumatoid arthritis, which is to be administered to patients with rheumatoid arthritis who have not been treated with disease modifying antirheumatic drugs (DMARDs) other than SMP-114 or have been treated with one or two DMARDs other than SMP-114.

22. The pharmaceutical composition according to claim 21, wherein the patients have been treated with one or two disease modifying antirheumatic drugs (DMARDs) other than SMP-114.

23. A pharmaceutical composition comprising SMP-114 or its pharmaceutically acceptable salt as the active ingredient and aiming at reduction of chronic inflammation, reduction of structural damage, reduction of progress of joint deformity or improving physical functions in patients with rheumatoid arthritis, which is to be administered to patients with rheumatoid arthritis having a physician's global assessment of less than 63 as a VAS (visual analogue scale) score.

24. A pharmaceutical composition comprising SMP-114 or its pharmaceutically acceptable salt as the active ingredient and aiming at reduction of chronic inflammation, reduction of structural damage, reduction of progress of joint deformity or improving physical functions in patients with rheumatoid arthritis, which is to be administered to patients with rheumatoid arthritis having a patient's assessment of pain of less than 67 as a VAS (visual analogue scale) score.
